# EUROPÄISCHE PATENTSCHRIFT

(11) **EP 3 221 326 B1**
(45) Veröffentlichungstag und Bekanntmachung des Hinweises auf die Patenterteilung: **01.05.2024**
(21) Anmeldenummer: 15791601.6
(22) Anmeldetag: 09.11.2015
(51) Int. Cl.: C07H 13/06, C11D 1/83

(54) **KONZENTRIERTE, NIEDRIGVISKOSE RHAMNOLIPID-ZUSAMMENSETZUNGEN**
CONCENTRATED LOW-VISCOSITY RHAMNOLIPID COMPOSITIONS
COMPOSITIONS DE RHAMNOLIPIDE CONCENTRÉES ET DE FAIBLE VISCOSITÉ

(30) Priorität: 19.11.2014 EP 14193779
(43) Veröffentlichungstag der Anmeldung: 27.09.2017
(73) Patentinhaber: Evonik Operations GmbH, 45128 Essen (DE)
(72) Erfinder: SCHILLING, Martin, 53121 Bonn (DE); KLEINEN, Jochen, 52525 Heinsberg (DE); LORENZ, Josef, 47809 Krefeld (DE); WENK, Hans Henning, 45470 Mülheim an der Ruhr (DE)
(74) Vertreter: Evonik Patent Association
(86) Internationale Anmeldenummer: PCT/EP2015/076019
(87) Internationale Veröffentlichungsnummer: WO 2016/078947

(56) Entgegenhaltungen:
- WO-A1-2006/096912
- WO-A1-2006/096912
- DE-A1-102012 221 519
- JP-A- S6 377 535
- JP-A- S6 377 535
- TW-A- 201 009 080
- TW-A- 201 009 080
- DATABASE CA [Online] CHEMICAL ABSTRACTS SERVICE, COLUMBUS, OHIO, US; 1989, SUZUKI, SHIGERU ET AL: "Synthesis and fluorescence properties of amphipathic fluorescent dye derived from microbial biosurfactants", XP002792578, gefunden im STN Database accession no. 1990:236889 & SUZUKI, SHIGERU ET AL: "Synthesis and fluorescence properties of amphipathic fluorescent dye derived from microbial biosurfactants", SHIKIZAI KYOKAISHI , 62(10), 594-9 CODEN: SKYOAO; ISSN: 0010-180X, 1989, DOI: 10.4011/SHIKIZAI1937.62.594 10.4011/SHIKIZAI1937.62.594
- DATABASE CA [Online] CHEMICAL ABSTRACTS SERVICE, COLUMBUS, OHIO, US; 1990, GAMA, YASUO ET AL: "Preparation of rhamnolipid pyrene acyl esters as fluorescent probes", XP002792579, gefunden im STN Database accession no. 1990:198973 & GAMA, YASUO ET AL: "Preparation of rhamnolipid pyrene acyl esters as fluorescent probes", JPN. KOKAI TOKKYO KOHO, 3 PP. CODEN: JKXXAF, 1990,
- YUTAKA ISHIGAMI ET AL: "Colloid chemical effect of polar head moieties of a rhamnolipid-type biosurfactant", LANGMUIR, Bd. 9, Nr. 7, 1. Juli 1993 (1993-07-01), Seiten 1634-1636, XP055190601, ISSN: 0743-7463, DOI: 10.1021/la00031a006
- DATABASE CA [Online] CHEMICAL ABSTRACTS SERVICE, COLUMBUS, OHIO, US; 1990, GAMA, YASUO ET AL: "Preparation of rhamnolipid pyrene acyl esters as fluorescent probes", XP002792579, retrieved from STN Database accession no. 1990:198973 & GAMA, YASUO ET AL: "Preparation of rhamnolipid pyrene acyl esters as fluorescent probes", JPN. KOKAI TOKKYO KOHO, 3 PP. CODEN: JKXXAF, 1990,

## Beschreibung

### Gebiet der Erfindung

Gegenstand der Erfindung sind bestimmte Salze von Rhamnolipiden.

### Stand der Technik

Rhamnolipide sind grenzflächenaktive Glykolipide und Stoffwechselprodukte bestimmter Mikroorganismen. Sie verfügen über besondere tensidische Eigenschaften, wie z.B. starke Schaumbildung und sind für verschiedenste, technische Anwendungen interessant. Rhamnolipide können sowohl mit Wildstämmen als auch durch gentechnisch veränderte Mikroorganismen hergestellt werden. Verfahren zur fermentativen Herstellung und Aufarbeitung von Rhamnolipiden sind im Stand der Technik ausführlich beschrieben, so z.B. in der US20130130319.

Tenside, die in kosmetische Formulierungen (z.B. Shampoos, Handwaschmittel), Haushaltsreiniger sowie Geschirrspülmittel eingearbeitet werden, müssen bei gängigen Verarbeitungstemperaturen in flüssiger Form vorliegen, um die Verarbeitung über Rohrleitungssysteme und Pumpen beim Hersteller solcher Produkte zu gewährleisten. Die Viskosität sollte hierbei gering sein, um eine einfache und zuverlässige Förderung sicherzustellen. Gleichzeitig ist hierbei trotzdem eine möglichst hohe Tensidkonzentration erwünscht, um umweltfreundliche Formulierungen mit einem geringen Wassergehalt herstellen zu können. Gleichzeitig soll der Viskositätsabfall bei Verdünnung mit Wasser so gering wie möglich gehalten werden, weil die formulierten Endprodukte wie z.B. Shampoos, Handwaschmittel und Waschmittel eine ausreichende Viskosität aufweisen sollen, um für den Anwender besser handhabbar zu sein.

Aus der Literatur sind keine Rhamnolipid enthaltenden Zusammensetzungen bekannt, die diesen Anforderungen entsprechen.

Bei den aus der Literatur bekannten und den auf dem Markt verfügbaren Produktformen, handelt es sich entweder um Feststoffe die über verschiedene Verfahren in mehr oder weniger reiner Form isoliert wurden (z.B. Entfernung eines Lösungsmittels durch Trocknung), um reine Produkte, die in geringer und mittlerer wässriger Konzentration vermarktet werden (z.B. bis 25 Gew. %), oder um flüssige Produkte bei denen die Hauptkomponente(n) aus der Fermentation stammendes Pflanzenöl bzw. Abbauprodukte des Pflanzenöls ist/sind. Bei diesen handelt es sich somit um Rhamnolipid Lösungen in einer Ölphase, und die entsprechenden Produkte haben den entscheidenden Nachteil, dass sie nur noch sehr schlecht schäumen und somit für eigentliche tensidische Anwendungen uninteressant sind.

Aus der DE4237334A1 ist weiterhin eine hochkonzentrierte Produktform bekannt, die durch ein besonders einfaches Verfahren zur Aufreinigung und Aufkonzentrierung von Rhamnolipiden erhalten werden kann. Hierbei wird das RL durch Ansäuern auf pH =3 aus einer Fermentationsbrühe ausgefällt und der entsprechende RL Feststoff kann durch Zentrifugation aufkonzentriert werden. Hierbei werden Feststoffsuspensionen bzw. Pasten mit einem hohen Rhamnolipid-Feststoffgehalt von 30 bis 40 Gew.-% und sehr hoher Viskosität erhalten.

Abdel-Mawgoud et al. in Appl Biochem Biotechnol. 2009 May;157(2):329-45 beschreiben ein ähnliches Verfahren.

Die derart erhaltenen Feststoffdispersionen sind für eine industrielle, großtechnische Weiterverarbeitung, wie z.B. Herstellung von Formulierungen für Shampoos und Haushaltsreiniger ungeeignet, da sie sich für eine Förderung mit üblicherweise eingesetzten Pumpen aufgrund ihrer hohen Viskosität und ihrer Inhomogenität nicht eignen.

DE102012221519A1 beschreibt ein Verfahren zur Gewinnung von Rhamnolipiden mit hervorragenden Schaumeigenschaften, bei dem wässrige Rhamnolipidlösungen mit einem pH-Wert von 7 und einer Konzentration von ca. 50 Gew.-% erhalten werden. Ein Nachteil diese Produktform ist der unerwünschte, sehr starke Viskositätsabfall bei Verdünnung. Ein weiterer Nachteil ist die im Neutralen verringerte mikrobiologische Stabilität

Aufgabe der Erfindung war es, hochkonzentrierte und niedrigviskose Rhamnolipid enthaltende Zusammensetzungen bereitzustellen. Diese sollen weiterhin eine möglichst niedrige lonenstärke aufweisen und eine Verdünnung mit Wasser soll zu möglichst geringen Viskositätsänderungen führen.

### Beschreibung der Erfindung

Überraschenderweise wurde gefunden, dass besonders hochkonzentrierte und gleichzeitig niedrigviskose Rhamnolipid enthaltende Zusammensetzungen durch eine teilweise Neutralisation einer hochkonzentrierten und hochviskosen, sauren Rhamnolipid Feststoffsuspension/paste zu erhalten sind. Diese Zusammensetzungen zeigen überraschenderweise bei Verdünnung mit Wasser einen geringeren Viskositätsabfall als vollständig neutralisierte Zusammensetzungen. Für die Neutralisation können anorganische und organische Basen eingesetzt werden. Gegenstand der vorliegenden Erfindung sind somit Salze mindestens eines Rhamnolipides, dadurch gekennzeichnet, dass es mindestens ein Kation enthält, ausgewählt aus der Gruppe bestehend aus, Li⁺, NH₄⁺, primäre Ammoniumionen, sekundäre Ammoniumionen, tertiäre Ammoniumionen und quaternäre Ammoniumionen.

Ein Vorteil der vorliegenden Erfindung ist, dass die lonenstärke der Rhamnolipid enthaltenden Zusammensetzungen klein gehalten werden kann. Hierdurch wird dem Formulierer entsprechende Flexibilität bei der nachträglichen Anpassung des pH-Wertes und des Salzgehaltes der finalen Endformulierung gelassen.

Ein weiterer Vorteil der vorliegenden Erfindung ist, dass die Zusammensetzungen eine erhöhte mikrobiologische Stabilität aufweisen.

Noch ein Vorteil der vorliegenden Erfindung ist, dass die Zusammensetzungen einfacher verdünnbar sind.

Ein weiterer Vorteil der vorliegenden Erfindung ist, dass die Zusammensetzungen mit anderen Tensiden gut mischbar sind.

Noch ein Vorteil der vorliegenden Erfindung ist, dass die Zusammensetzungen die Formulierung konzentrierter Tensidendformulierungen ("*concentrates*") ermöglichen. Ein weiterer Vorteil der vorliegenden Erfindung ist, dass die Zusammensetzungen aufgrund ihrer hohen Konzentration eine verringerte Schaumtendenz aufweisen, und somit Transport und Förderung vereinfacht werden.

Noch ein Vorteil der vorliegenden Erfindung ist, dass die Zusammensetzungen eine einfache Einarbeitung hydrophober Komponenten wie z. B. Öle erlauben.

Ein weiterer Vorteil der vorliegenden Erfindung ist, dass die Zusammensetzungen eine hohe Lagerstabiliät aufweisen.

Noch ein Vorteil der vorliegenden Erfindung ist, dass die Zusammensetzungen bei Verdünnung mit Wasser keine extremen Viskositätssprünge durchlaufen.

Ein weiterer Vorteil der vorliegenden Erfindung ist, dass die Zusammensetzungen bei ihrer Herstellung und ihrem Transport in Rohrleitung geringere Verschmutzungen verursachen und überdies hinaus eine einfachere Reinigung ermöglichen.

Noch ein Vorteil der vorliegenden Erfindung ist, dass die Zusammensetzungen zu ihrem Transport einen geringeren Energiebedarf zum Transport erfordern.

Unter dem Begriff "Rhamnolipid" im Zusammenhang mit der vorliegenden Erfindung werden Rhamnolipide, deren protonierten Formen sowie insbesondere deren Salze mitumfasst.

Unter dem Begriff "Rhamnolipid" im Zusammenhang mit der vorliegenden Erfindung werden insbesondere Mischungen aus Verbindungen der allgemeinen Formel (I) und deren Salze verstanden, 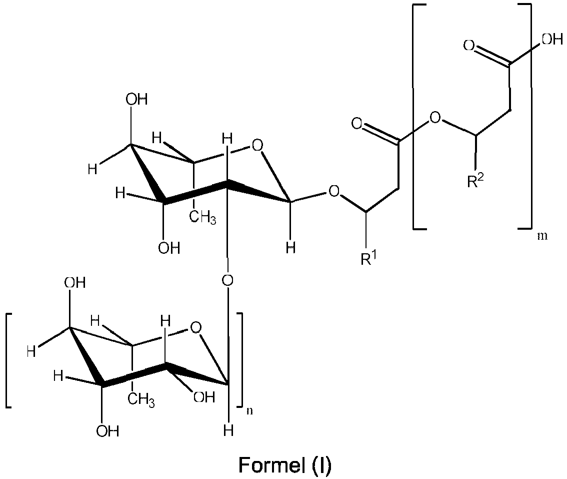 wobei
m = 2, 1 oder 0,
n = 1 oder 0,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH₂)ₒ-CH₃ mit o = 1 bis 23, bevorzugt 4 bis 12.
Falls n = 1 ist die glykosidische Bindung zwischen den zwei Rhamnoseeinheiten bevorzugt in der α-Konfiguration. Die optisch aktiven Kohlenstoffatome der Fettsäuren liegen bevorzugt als R-Enantiomere vor (z.B. (R)-3-{(R)-3-[2-O-(α-L-rhamnopyranosyl)-α-L-rhamnopyranosyl]oxydecanoyl}oxydecanoate).

Unter dem Begriff "di-Rhamnolipid" im Zusammenhang mit der vorliegenden Erfindung werden Verbindungen der allgemeinen Formel (I) oder deren Salze verstanden, bei denen n = 1.

Unter dem Begriff "mono-Rhamnolipid" im Zusammenhang mit der vorliegenden Erfindung werden Verbindungen der allgemeinen Formel (I) oder deren Salze verstanden, bei denen n = 0.

Distinkte Rhamnolipide werden gemäß folgender Nomenklatur abgekürzt:
Unter "diRL-CXCY" werden di-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Unter "monoRL-CXCY" werden mono-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Die verwendete Nomenklatur unterscheidet somit nicht zwischen "CXCY" und "CYCX". Für Rhamnolipide mit m=0 wird entsprechend monoRL-CX bzw. diRL-CX verwendet. Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.

Der "pH-Wert" im Zusammenhang mit der vorliegenden Erfindung ist definiert als der Wert, welcher für die entsprechende Zusammensetzung bei 25 °C nach fünf Minuten Rühren mit einer gemäß ISO 4319 (1977) kalibrierten pH-Elektrode gemessen wird.

Alle angegebenen Prozent (%) sind, wenn nicht anders angegeben, Massenprozent. Zur Bestimmung des Gehaltes an Rhamnolipiden im Zusammenhang mit der vorliegenden Erfindung wird lediglich die Masse des Rhamnolipid-Anions, somit "allgemeine Formel (I) abzüglich ein Wasserstoff" berücksichtigt.

Zur Bestimmung des Gehaltes an Rhamnolipiden im Zusammenhang mit der vorliegenden Erfindung werden alle Rhamnolipide durch Ansäuern in die protonierte Form (vgl. allgemeine Formel (I)) überführt und mittels HPLC quantifiziert.

Beansprucht wird ein Salz mindestens eines Rhamnolipides,
wobei das Rhamnolipid eine Mischung aus Verbindungen der allgemeinen Formel (I) ist, 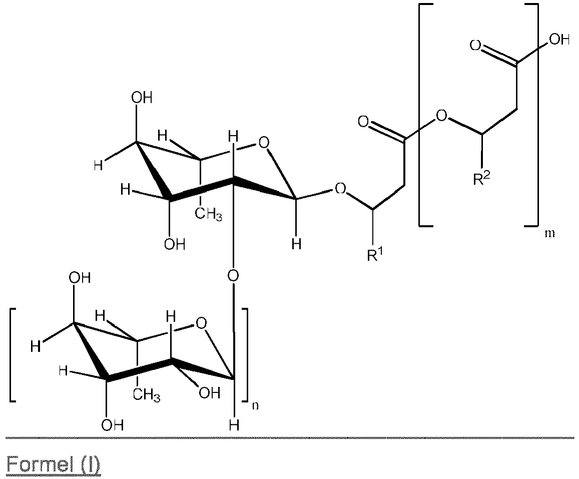
wobei
   m = 2, 1 oder 0,
   n = 1 oder 0,
   R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH2)ₒ-CH₃ mit o = 1 bis 23, bevorzugt 4 bis 12.
   dadurch gekennzeichnet
   dass es mindestens ein Kation enthält, ausgewählt aus der Gruppe bestehend aus Li⁺, NH₄+, primäre Ammoniumionen, sekundäre Ammoniumionen, tertiäre Ammoniumionen und quaternäre Ammoniumionen.

Bevorzugt enthalten die erfindungsgemäßen Salze zu mindestens 50 Gew.-%, bevorzugt mindestens 70 Gew.-%, besonders bevorzugt mindestens 95 Gew.-% das mindestens eine Kation, wobei die Gewichtsprozente sich aus dem Gewicht des Gesamtsalzes "Rhamnolipid-Anion plus Kation" ergeben und sich auf das gesamte Salz beziehen.

Beispielhafte Vertreter von geeigneten Ammoniumionen sind Tetramethylammonium, Tetraethylammonium , Tetrapropylammonium, Tetrabutylammonium und [(2-Hydroxvethyl)trimethylammoniuml (Cholin) sowie die Kationen von 2-Aminoethanol (Ethanolamin, MEA), Diethanolamin (DEA), 2,2',2"-Nitrilotriethanol (Triethanolamin, TEA), 1-Aminopropan-2-ol (Monoisopropanolamin), Ethylendiamin, Diethylentriamin, Triethylentetramin, Tetraethylenepentamine, 1,4-Diethylendiamin (Piperazin), Aminoethylpiperazin und Aminoethylethanolamin.

Besonders bevorzugte Kationen, sind ausgewählt aus der Gruppe bestehend aus NH₄⁺ und das Triethanolammonium-Kation.

Es ist erfindungsgemäß bevorzugt, dass die Salze 51 Gew.-% bis 95 Gew.-%, bevorzugt 70 Gew.-% bis 90 Gew.-%, besonders bevorzugt 75 Gew.-% bis 85 Gew.-%, diRL-C10C10 enthalten, wobei sich die Gewichtsprozente auf die Summe aller enthaltenen Rhamnolipide beziehen.

Es ist erfindungsgemäß bevorzugt, dass die Salze 0,5 Gew.-% bis 9 Gew.-%, bevorzugt 0,5 Gew.-% bis 3 Gew.-%, besonders bevorzugt 0,5 Gew.-% bis 2 Gew.-%, monoRL-C10C10 enthalten, wobei sich die Gewichtsprozente auf die Summe aller enthaltenen Rhamnolipide beziehen.

Bevorzugte erfindungsgemäße Salze sind dadurch gekennzeichnet, dass das Gewichtsverhältnis von allen enthaltenen di-Rhamnolipiden zu allen enthaltenen mono-Rhamnolipiden größer 51:49, insbesondere größer 91:9, bevorzugt größer 97:3, besonders bevorzugt größer 98:2, ist.

Es ist erfindungsgemäß bevorzugt, dass die Salze 0,5 bis 25 Gew.-%, bevorzugt 5 Gew.-% bis 15 Gew.-%, besonders bevorzugt 7 Gew.-% bis 12 Gew.-%, diRL-C10C12 enthalten, wobei sich die Gewichtsprozente auf die Summe aller enthaltenen Rhamnolipide beziehen.

Es ist erfindungsgemäß bevorzugt, dass die Salze 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und/oder, bevorzugt und, 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1, enthalten, wobei sich die Gewichtsprozente auf die Summe aller enthaltenen Rhamnolipide beziehen.

Besonders bevorzugte erfindungsgemäße Salze sind dadurch gekennzeichnet, dass sie
0,5 Gew.-% bis 15 Gew.-%, bevorzugt 3 Gew.-% bis 12 Gew.-%, besonders bevorzugt 5 Gew.-% bis 10 Gew.-%, diRL-C10C12:1,
0,5 bis 25 Gew.-%, bevorzugt 5 Gew.-% bis 15 Gew.-%, besonders bevorzugt 7 Gew.-% bis 12 Gew.-%, diRL-C10C12,
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthalten, wobei sich die Gewichtsprozente auf die Summe aller enthaltenen Rhamnolipide beziehen.

Es ist überdies hinaus bevorzugt, wenn das erfindungsgemäße Salz Rhamnolipide der Formel monoRL-CX bzw. diRL-CX in nur kleinen Mengen enthält. Insbesondere enthält das erfindungsgemäße Salz bevorzugt
0 Gew.-% bis 5 Gew.-%, bevorzugt 0 Gew.-% bis 3 Gew.-%, besonders bevorzugt 0 Gew.-% bis 1 Gew.-%, diRLC10, wobei sich die Gewichtsprozente auf die Summe aller enthaltenen Rhamnolipide beziehen, und unter dem Begriff "0 Gew.-%" keine nachweisbare Menge zu verstehen ist.

Die Salze der vorliegenden Erfindung lassen sich durch ein Verfahren zur Herstellung einer Lösung von Rhamnolipiden umfassend die Schritte
a) Bereitstellen einer Zusammensetzung enthaltend
   30 Gew.-% bis 70 Gew.-%, bevorzugt 35 Gew.-% bis 60 Gew.-%, besonders bevorzugt 40 Gew.-% bis 50 Gew.-%, mindestens eines Rhamnolipides wobei sich die Gewichtsprozente auf die Gesamtzusammensetzung beziehen, mit einem pH-Wert bei 25 °C von pH 1 bis pH 5, bevorzugt von pH 2,5 bis 4,0, besonders bevorzugt von pH 3,0 bis pH 3,5
b) Einstellen des pH-Wertes der Zusammensetzung auf von pH 5,5 bis pH < 7,0, bevorzugt auf von pH 5,6 bis pH 6,2, besonders bevorzugt auf von pH 5,6 bis pH 6,0 und
c) Einstellen des Wassergehaltes der Gesamtzusammensetzung auf 30 Gew.-% bis 70 Gew.-%, bevorzugt 40 Gew.-% bis 65 Gew.-%, besonders bevorzugt 50 Gew.-% bis 60 Gew.-%, Wasser

und des Rhamnolipidgehaltes der Gesamtzusammensetzung auf 30 Gew.-% bis 70 Gew.-%, bevorzugt 35 Gew.-% bis 60 Gew.-%, besonders bevorzugt 40 Gew.-% bis 50 Gew.-%, Rhamnolipide,
wobei sich die Gewichtsprozente auf die Gesamtzusammensetzung beziehen,
wobei in Verfahrensschritt b) der pH-Wert durch Zugabe einer organischen oder anorganischen Base, bevorzugt in konzentrierter Form, eingestellt wird,
wobei die Base ausgewählt ist aus der Gruppe umfassend, bevorzugt bestehend aus Alkali- und Erdalkalimetallhydroxiden wie NH₄OH, primäre Amine, sekundäre Amine, tertiäre Amine und quarternäre Amine,
herstellen.

Bevorzugt liegt das Rhamnolipid in der in Verfahrensschritt a) bereitgestellten Zusammensetzung mindestens teilweise dispers vor.

Unter dem Begriff "dispers vorliegen" im Zusammenhang mit der vorliegenden Erfindung, wird verstanden, dass visuell oder lichtmikroskopisch identifizierbare Rhamnolipidaggregate vorliegen, die bei Verdünnung mit Wasser unter Beibehaltung des pH-Wertes auf 10 Gew.-% Rhamnolipid im Schwerefeld der Erde sedimentieren.

Insbesondere werden in Verfahrensschritt a) Zusammensetzungen bereitgestellt, die eine Viskosität von 3 bis 15, bevorzugt von 5 bis 10, besonders bevorzugt von 6 bis 8 Pas, gemessen in einem Rheometer bei einer Scherrate von 10 s⁻¹, aufweisen.

Unter dem Begriff "Base in konzentrierter Form" im Zusammenhang mit der vorliegenden Erfindung, wird verstanden, dass die Base in Form einer Zusammensetzung zugegeben wird, die mindestens 60 Gew.-%, insbesondere mindestens 80 Gew.-% Base enthält, wobei sich die Gewichtsprozente auf die zugegebene Gesamtzusammensetzung beziehen.

In dem Verfahren werden bevorzugt Basen ausgewählt aus der Gruppe umfassend, bevorzugt bestehend aus 2-Aminoethanol (auch Ethanolamin, MEA), Diethanolamin (auch DEA), 2,2',2"-Nitrilotrietanol (auch Triethanolamin, TEA), 1-Aminopropan-2-ol (auch Monoisopropanolamin), [(2-Hydroxyethyl)trimethylammonium] (auch Cholin) Ethylendiamin, Diethylentriamin, Triethylentetramin, Tetraethylenepentamine, 1,4-Diethylendiamin (auch Piperazin), Aminoethylpiperazin, Aminoethylethanolamin, , Tetramethylammoniumhydroxid, Tetraethylammoniumhydroxid , Tetrapropylammoniumhydroxid, Tetrabutylammoniumhydroxid, wobei 2-Aminoethanol (auch Ethanolamin, MEA), Diethanolamin (auch DEA), 2,2',2"-Nitrilotrietanol (auch Triethanolamin, TEA), 1-Aminopropan-2-ol (auch Monoisopropanolamin) und (2-Hydroxyethyl)trimethylammonium (auch Cholin) eingesetzt.

Besonders bevorzugte Basen sind NH₃. NH₄OH und Triethanolamin. Es können auch Mischungen der vorgenannten Basen eingesetzt werden.

Aus den erfindungsgemäßen Lösungen erhältlich nach dem Verfahren lassen sich durch beispielsweise Wasserentfernung die erfindungsgemäßen Salze von Rhamnolipiden isolieren.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Charakterisierung der Produkte

### Quantifizierung von Rhamnolipiden mittels Hochleistungsflüssigchromatographie (HPLC)

Die Quantifizierung erfolgte mittels HPLC. Als Kalibrierstandard wurde jeweils die Säureform der verschiedenen Rhamnolipidspezies verwendet, die chromatographische Auftrennung erfolgte ebenfalls unter sauren Bedingungen. Dies führt dazu, dass alle RL Komponenten in eine Probe als Säureform quantifiziert werden.

Für die Probenvorbereitung wurde mit einer Verdrängerpipette (Combitip) in einem 2 ml-Reaktionsgefäß 1 ml Aceton vorgelegt und das Reaktionsgefäß zur Minimierung von Verdunstung unmittelbar verschlossen. Es folgte die Zugabe von 1 ml Rhamnolipid-haltiger Probe, die zuvor nach Bedarf verdünnt worden war. Nach Vortexen wurde die Mischung für 3 min bei 13.000 rpm abzentrifugiert, und 800 µl des Überstands in ein HPLC-Gefäß überführt.

Zum Nachweis und zur Quantifizierung von Rhamnolipiden wurde ein Evaporative Light Scattering Detektor (Sedex LT-ELSD Model 85LT) benutzt. Die eigentliche Messung wurde mittels Agilent Technologies 1200 Series (Santa Clara, Kalifornien) und der Zorbax SB-C8 Rapid Resolution Säule (4,6 x 150 mm, 3,5 µm, Agilent) durchgeführt. Das Injektionsvolumen betrug 5 µl und die Laufzeit der Methode 20 min. Als mobile Phase wurde wässrige 0,1 % TFA (Trifluor-Essigsäure, Lösung A) und Methanol (Lösung B) verwendet. Die Säulentemperatur betrug 40 °C. Als Detektoren dienen der ELSD (Detektortemperatur 60 °C) und der DAD (Diodenarray, 210 nm). Der in der Methode verwendete Gradient war:

| **t [min]** | **Lösung B vol.-%** | **Fluss [ml/min]** |
|---|---|---|
| 0,00 | 70% | 1,00 |
| 15,00 | 100% | 1,00 |
| 15,01 | 70% | 1,00 |
| 20,00 | 70% | 1,00 |

### Viskositätsmessungen

Die Messung der Viskosität erfolgt mit Hilfe eines Rheometers (MCR 302, Anton Paar Germany) in einem Platte-Platte Messsystem. Die obere Platte hatte einen Durchmesser von 40 mm, der Spaltabstand betrug 0,5 mm, Messtemperatur war 25°C. Es wurde im Scherratenbereich von 0.1 -100 s-1 gemessen.

### Beispiel 1: Herstellung einer hochkonzentrierten RL-Lösung:

Eine Fermentation mit einem die Rhamnolipid-Biosynthesegene RhIA, RhIB und RhIC enthaltenden *Pseudomonas putida* Stamm *pBBR1MCS2-Plac-rhlABC-T-Ptac-rhlC-T,* deren Herstellung in US2014296168 beschrieben ist, wurde durchgeführt. Die Vorkultur im Schüttelkolben wurde wie in WO2012013554A1 beschrieben durchgeführt. Für die Hauptkultur kam ebenfalls ein Mineralmedium (M9) zum Einsatz.

Die Fermentation wurde kohlenstofflimitiert über eine Glukosezufütterung in einem 2 Liter Fermenter geführt. Die Glukosezufütterung erfolgt anhand des Gelöstsauerstoffsignals. Der Gelöstsauerstoff wurde bei 20 % Sättigung über die Rührerdrehzahl reguliert. Der pH-Wert wird über eine pH Elektrode und Zugabe von NH₄SO₄ auf 7 reguliert. Um das Überschäumen der Fermentationsbrühe zu verhindern, wurde der Entschäumer DOW Corning 1500 bei Bedarf zudosiert. Die Fermentation wurde über 4 Tage bis zu einer Biotrockenmasse von 15 g/l geführt. Die Rhamnolipidkonzentration wurde über HPLC ermittelt und betrug 9,8 g/l. Nach Abtrennen der Zellen mittels Zentrifugation bei 10.000 g wurde die Fermentationsbrühe durch Zugabe konzentrierter H₂SO₄ auf einen pH-Wert von 3,1 eingestellt. Durch erneute Zentrifugation bei 500 g wurde ein pastöses Feststoff-Konzentrat mit einem RL-Anteil von 45 Gew. % und mit einer Viskosität von > 10.000 mPas erhalten.

### Beispiel 2 (nicht erfindungsgemäß): Teilneutralisation mit KOH:

Unter ständigem Rühren wurde eine 50 gew.-%ige wässrige KOH Lösung zur pastösen Suspension des aufkonzentrierten Rhamnolipidpräzipitats (vgl. vorheriges Beispiel) gegeben und ein pH-Wert von 5,6 eingestellt. Hierbei kam es zur Verflüssigung der pastösen Masse die mit einem starken Viskositätsabfall einherging. Aus der Suspension wurde eine klare Lösung. Von dieser Lösung wurde 3 Teile abgenommen und durch weitere KOH Zugabe jeweils auf pH = 6, pH = 7 und pH = 8,5 eingestellt. Nach Einstellung der pH-Werte der Lösungen wurde diese durch Wasserzugabe auf 50 Gew %, 40 Gew %, 30 Gew.%, 20 Gew% und 10 Gew% Rhamnolipid eingestellt und für Viskositätsmessungen verwendet. Die Ergebnisse sind in Tabelle 1 dargestellt. Es wurde festgestellt, dass durch die Teilneutralisation ein wesentlich dünnflüssigeres, aber hochkonzentriertes Produkt erhalten werde konnte. Gleichzeitig kam es bei den pH-Werten 5,6 und 6 zu einem wesentlich geringeren Viskositätsabfall beim Verdünnen der Lösungen als bei den pH-Werten 7 und 8,5.

**Tabelle 1: Viskosität (Pas, Scherrate 10 1/s) von Rhamnolipidzusammensetzungen in Abhängigkeit von Rhamnolipidkonzentration (Gew. %) und pH-Wert. Die durch * gekennzeichneten Formulierungen sind erfindungsgemäß, da hierbei die Viskosität bei hoher Konzentration gering ist und der Viskositätsabfall bei Verdünnung mit Wasser ebenfalls am geringsten ist (vergleiche die jeweiligen Viskositäten bei 10 % und 20 %).**

| | 10% | 20% | 30% | 40% | 50% |
|---|---|---|---|---|---|
| pH 3,5 | - | - | - | 6,04 | |
| pH 5,6 | 0,114 | 0,406 | 0,593* | 0,816* | 1,756* |
| pH 6,0 | 0,064 | 0,12 | 0,536* | 0,922* | 1,698* |
| pH 7,0 | 0,001 | 0,014 | 0,212 | 1,010 | 1,793 |
| pH 8,5 | 0,002 | 0,009 | 0,135 | 0,827 | 1,728 |

### Beispiel 3 (nicht erfindungsgemäß): Teilneutralisation mit NaOH:

Die Teilneutralisation erfolgte analog zu Beispiel 2, mit der Ausnahme, dass Anstelle von KOH eine 50 Gew. %ige NaOH Lösung verwendet wurde.

### Beispiel 4: Teilneutralisation mit NH₄OH:

Die in Beispiel 1 erhaltene, hochkonzentrierte, saure Rhamnolipidsuspension wurde durch Zugabe von Wasser auf einen Rhamnolipidgehalt von 34,6 Gew.% eingestellt, die einen pH-Wert von 3,18 hatte. Es wurden 113 g dieser Suspension entnommen und unter ständigem Rühren wurden 3,64 g einer 25 Gew. %igen NH₄OH Lösungen zugegeben. Hierbei kam es zu einer vollständigen Verflüssigung und es stellte sich ein pH-Wert von 5,63 ein.

### Beispiel 5(nicht erfindungsgemäß): Teilneutralisation mit Ca(OH)₂

Zu 101 g einer sauren Rhamnolipidsuspension mit 34,6 Gew.% Rhamnolipid und einem pH-Wert von 3,18 wurden 2 g Ca(OH)₂ gegeben und die resultierende Feststoffsuspension 2 Tage bei Raumtemperatur gerührt. Hierbei kam es zu einer vollständigen Verflüssigung und es stellte sich ein pH-Wert von 5,72 ein.

### Beispiel 6 (nicht erfindungsgemäß): Teilneutralisation mit Mg(OH)₂

Zu 95 g einer sauren Rhamnolipidsuspension mit 34,6 Gew.% Rhamnolipid und einem pH-Wert von 3,18 wurden 1,69 g Mg(OH)₂ gegeben und die resultierende Feststoffsuspension über Nacht bei Raumtemperatur gerührt. Hierbei kam es zu einer vollständigen Verflüssigung und es stellte sich ein pH-Wert von 6,06 ein.

### Beispiel 7: Teilneutralisation mit 2-Aminoethanol (MEA)

Die Fermentation und Aufarbeitung der Rhamnolipide wurde analog zu Beispiel 1 durchgeführt, mit der Ausnahme, dass für den Fällungsschritt lediglich auf pH = 3,8 angesäuert wurde. Die erhaltene Rhamnolipidsuspension wurde durch Wasserzugabe auf einen Rhamnolipidgehalt von 40 Gew.% eingestellt und in mehrer Teile von 50 g aufgeteilt. Dann wurden unter ständigem Rühren 2,28 g Monoethanolamin (Reinheit > 99 %) zu einem der Teile zugegeben, was zu einer Verflüssigung und zum Aufklaren der Lösung führte. Der pH-Wert der erhaltenen Lösung war 6,0.

### Beispiel 8: Teilneutralisation mit Triethanolamin (TEA)

Zu 50 g einer 40 Gew.% Rhamnolipidsuspension, pH = 3,8 (vgl. Beispiel 7) wurden unter ständigem Rühren 6,8 g Triethanolamin (Reinheit > 99 %) gegeben. Dies führte zur Verflüssigung zum Aufklaren der Lösung. Der pH-Wert der erhaltenen Lösung betrug 6,2.

### Beispiel 9: Teilneutralisation mit Monoisopropanolamin (MIPA)

Zu 50 g einer 40 Gew.% Rhamnolipidsuspension, pH = 3,8 (vgl. Beispiel 7) wurden unter ständigem Rühren 2,76 g MIPA (Reinheit > 99 %)gegeben. Dies führte zur Verflüssigung zum Aufklaren der Lösung. Der pH-Wert der erhaltenen Lösung betrug 6,2.

### Beispiel 10: Teilneutralisation mit Cholinhydroxid

Zu 50 g einer 40 Gew.% Rhamnolipidsuspension, pH = 3,8 (vgl. Beispiel 7) wurden unter ständigem Rühren 9,4 g Cholinhydroxid (46 Gew.% Lösung) gegeben. Dies führte zur Verflüssigung zum Aufklaren der Lösung. Der pH-Wert der erhaltenen Lösung betrug 6,2.

## Patentansprüche

1. Salz mindestens eines Rhamnolipides,
wobei das Rhamnolipid eine Mischung aus Verbindungen der allgemeinen Formel (I) ist, 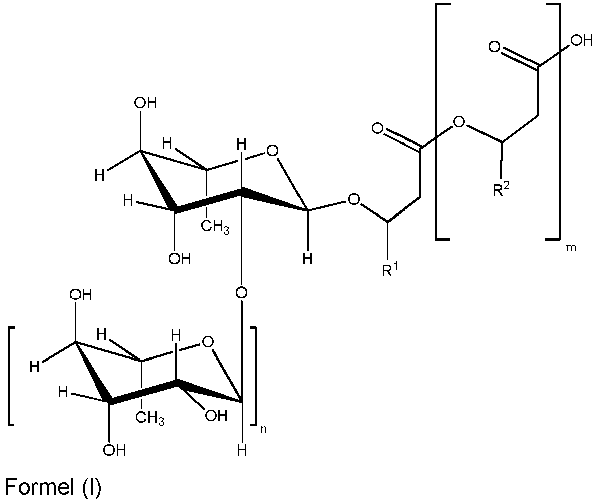
wobei
m = 2, 1 oder 0,
n = 1 oder 0,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH₂)ₒ-CH₃ mit o = 1 bis 23, bevorzugt 4 bis 12.
**dadurch gekennzeichnet**
**dass** es mindestens ein Kation enthält, ausgewählt aus der Gruppe bestehend aus Li⁺, NH₄⁺, primäre Ammoniumionen, sekundäre Ammoniumionen, tertiäre Ammoniumionen und quaternäre Ammoniumionen.

2. Salz nach Anspruch 1, **dadurch gekennzeichnet, dass** das mindestens eine Kation ausgewählt ist aus der Gruppe bestehend aus Li⁺, NH₄⁺ und das Triethanolammonium-Kation.

3. Salz nach Anspruch 1, **dadurch gekennzeichnet, dass** das mindestens eine Kation ausgewählt ist aus der Gruppe bestehend aus Tetramethylammonium, Tetraethylammonium, Tetrapropylammonium, Tetrabutylammonium und [(2-Hydroxyethyl)trimethylammonium] sowie die Kationen von 2-Aminoethanol, Diethanolamin, 2,2',2"-Nitrilotriethanol, 1-Aminopropan-2-ol, Ethylendiamin, Diethylentriamin, Triethylentetramin, Tetraethylenepentamine, 1,4-Diethylendiamin, Aminoethylpiperazin und Aminoethylethanolamin.

## Claims

1. Salt of at least one rhamnolipid,
wherein the rhamnolipid is a mixture of compounds of the general formula (I), 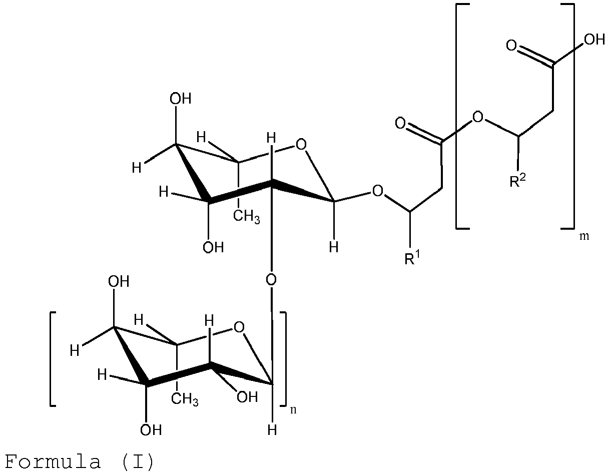
wherein
m = 2, 1 or 0,
n = 1 or 0,
R¹ and R² = mutually independently, identical or different, organic radicals having 2 to 24, preferably 5 to 13 carbon atoms, in particular optionally branched, optionally substituted, particularly hydroxy-substituted, optionally unsaturated, in particular optionally mono-, bi- or tri-unsaturated alkyl radicals, preferably those selected from the group consisting of pentenyl, heptenyl, nonenyl, undecenyl and tridecenyl and (CH₂)ₒ-CH₃ where o = 1 to 23, preferably 4 to 12, **characterized in that**
said rhamnolipid comprises at least one cation selected from the group consisting of Li⁺, NH₄⁺, primary ammonium ions, secondary ammonium ions, tertiary ammonium ions and quaternary ammonium ions.

2. Salt according to Claim 1, **characterized in that** the at least one cation is selected from the group consisting of Li⁺, NH₄⁺ and the triethanolammonium cation.

3. Salt according to Claim 1, **characterized in that** the at least one cation is selected from the group consisting of tetramethylammonium, tetraethylammonium, tetrapropylammonium, tetrabutylammonium and [(2-hydroxyethyl)trimethylammonium] and also the cations of 2-aminoethanol, diethanolamine, 2,2',2"-nitrilotriethanol, 1-aminopropan-2-ol, ethylenediamine, diethylenetriamine, triethylenetetramine, tetraethylenepentamine, 1,4-diethylenediamine, aminoethylpiperazine and aminoethylethanolamine.

## Revendications

1. Sel d'au moins un rhamnolipide
le rhamnolipide étant un mélange de composés de formule générale (I), 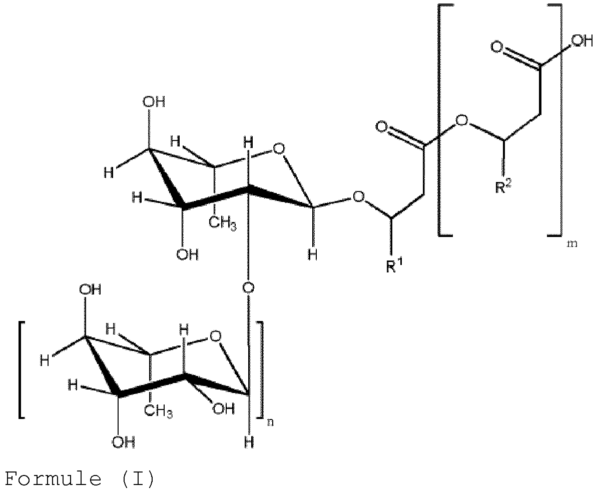
dans laquelle
m = 2, 1 ou 0,
n = 1 ou 0,
R¹ et R² = indépendamment l'un de l'autre un radical organique identique ou différent ayant de 2 à 24, de préférence 5 à 13 atomes de carbone, en particulier un radical alkyle éventuellement ramifié, éventuellement substitué, en particulier substitué par hydroxy, éventuellement insaturé, en particulier éventuellement une fois, deux fois ou trois fois insaturé, de préférence un tel radical alkyle choisi dans le groupe constitué par les groupes pentényle, heptényle, nonényle, undécényle et tridécényle et (CH₂)ₒ-CH₃ où o = 1 à 23, de préférence 4 à 12,
**caractérisé en ce**
**qu'**il contient au moins un cation, choisi dans le groupe constitué par Li⁺, NH₄⁺, les ions ammonium primaires, les ions ammonium secondaires, les ions ammonium tertiaires et les ions ammonium quaternaires.

2. Sel selon la revendication 1, **caractérisé en ce que** ledit au moins un cation est choisi dans le groupe constitué par Li⁺, NH₄⁺ et le cation triéthanolammonium.

3. Sel selon la revendication 1, **caractérisé en ce que** ledit au moins un cation est choisi dans le groupe constitué par les cations tétraméthylammonium, tétraéthylammonium, tétrapropylammonium, tétrabutyl-ammonium et [(2-hydroxyéthyl)triméthylammonium] ainsi que les cations de 2-aminoéthanol, diéthanolamine, 2,2',2"-nitrilotriéthanol, 1-aminopropan-2-ol, éthylènediamine, diéthylènetriamine, triéthylène-tétramine, tétraéthylènepentamine, 1,4-diéthylène-diamine, aminoéthylpipérazine et aminoéthyl-éthanolamine.
